# EUROPÄISCHE PATENTSCHRIFT

(11) **EP 1 337 651 B1**
(45) Veröffentlichungstag und Bekanntmachung des Hinweises auf die Patenterteilung: **08.02.2006**
(21) Anmeldenummer: 01990387.1
(22) Anmeldetag: 03.11.2001
(51) Int. Cl.: C12N 15/81

(54) **PROMOTOR ZUR FUNKTIONELLEN CHARAKTERISIERUNG VON G-PROTEIN GEKOPPELTEN REZEPTOREN IN DER HEFE SACCHAROMYCES CEREVISIAE**
PROMOTER FOR THE FUNCTIONAL CHARACTERISATION OF G-PROTEIN COUPLED RECEPTORS IN THE YEAST SACCHAROMYCES CEREVISIAE
PROMOTEUR POUR LA CARACTERISATION FONCTIONNELLE DE RECEPTEURS COUPLES AUX PROTEINES G DANS LA LEVURE SACCHAROMYCES CEREVISIAE

(30) Priorität: 16.11.2000 DE 10056899
(43) Veröffentlichungstag der Anmeldung: 27.08.2003
(73) Patentinhaber: Sanofi-Aventis Deutschland GmbH, 65929 Frankfurt am Main (DE)
(72) Erfinder: KOZIAN, Detlef, 65779 Kelkheim (DE); NITSCHE, Almut, 65185 Wiesbaden (DE); NOACK, Pauline, 76120 Grand Quevilly (FR); GRATZER, Sabine, 65812 Bad Soden (DE); LEBERER, Ekkehard, 82100 Germering (DE)
(86) Internationale Anmeldenummer: PCT/EP2001/012748
(87) Internationale Veröffentlichungsnummer: WO 2002/040660

(56) Entgegenhaltungen:
- WO-A-00/12704
- WO-A-01/16378
- WO-A-99/51620
- US-A- 5 063 154
- PAUSCH M H: "G-protein-coupled receptors in Saccharomyces cerevisiae: high-throughput screening assays for drug discovery" TRENDS IN BIOTECHNOLOGY, ELSEVIER PUBLICATIONS, CAMBRIDGE, GB, Bd. 15, Nr. 12, 1. Dezember 1997 (1997-12-01), Seiten 487-494, XP004097426 ISSN: 0167-7799
- DATABASE EMBL [Online] 6. Mai 1996 (1996-05-06) retrieved from EBI Database accession no. Z71555 XP002217914

### SEQUENZPROTOKOLL

<110> Aventis Pharma Deutschland GmbH
<120> Promotor zur funktionellen Charakterisierung von G-Protein gekoppelten Rezeptoren in der Hefe Saccharomyces cerevisiae
<130> D-2000/A049
<140> PCT/EP 01/12749
   <141> 2001-11-03
<150> 10056899.8
   <151> 2000-11-16
<160> 4
<170> Patent In Ver. 2.1
<210> 1
   <211> 30
   <212> DNA
   <213> Künstliche Sequenz
<220>
   <221> misc feature
   <222> (1)..(30)
<220>
   <223> Beschreibung der küns Sequenz:Oligonukleoti
<400> 1
   ccgagtccta ctcctatgct gtttacaagg 30
<210> 2
   <211> 31
   <212> DNA
   <213> Künstliche Sequenz
<220>
   <221> misc_feature
   <222> (1)..(31)
<220>
   <223> Beschreibung der künstlichen Sequenz:Oligonukleotid
<400> 2
   tgctctagaa tcatcaacgt tcacaaattc g 31
<210> 3
   <211> 2860
   <212> DNA
   <213> Saccharomyces cerevisiae
<400> 3
<210> 4
   <211> 670
   <212> DNA
   <213> Saccharomyces cerevisiae
<400> 4
   ccttttattc tctacaccga atttgtcttt actcctatgc tgtttacaag gtctatctga 60
   taagcaattg cgcaagaaaa tagtagaatg aaaactgatt attaaaaaca aacgtaaact 120
   caagcctcac ttgatgctca gacggagtac gtgaaaaacg tccgttatgc aaaacccttt 180
   atatgcacaa ccttcacaca atgcaaattt ccgatgatgc ctacatacaa aagagcgaaa 240
   ggcgatataa atttttttca cgggattttc gtttaggtga aaataaaatg aacgacagag 300
   catgcagagt ccgggtaata catatgtttc aatactgttt caatactgtt tcagaagtgc 360
   gtcacatatt aattttaact tataactggc ctgttgctgg caagaggtat atatatatga 420
   cgaatgtgac caacataagt ccttaagata atcccgaaat atttggttag gatgattccc 480
   tttcgaattt gtgaacgttg atgatatgag cggttttaaa tgctatttgc aattgggtga 540
   caggctctct caaatabbdn gctgtttcac nsnsssnmsm atchtgaaaa anvrsrntrn 600
   grcnsnsssn tttttcacns nsssntgttt cacnsnsssn tgtttcacns nsssntgttt 660
   cacnsnsssn                                                        670

## Beschreibung

Die vorliegende Erfindung betrifft einen Promotor, DNA Fragmente, die diesen Promotor enthalten sowie deren Verwendung, insbesondere in Verfahren zur Identifizierung von Substanzen, die auf G-Protein gekoppelte Rezeptoren eine aktivierende oder inhibierende Wirkung zeigen.

GPCRs (G-Protein gekoppelte Rezeptoren) bilden eine Genfamilie strukturell und funktionell verwandter Transmembranproteine. Für die medizinische Forschung und Entwicklung pharmakologischer Wirksubstanzen sind GPCRs Zielmoleküle von großer Bedeutung und nehmen in einer Vielzahl von Pathologien eine Schlüsselstellung ein (Stadel et al., 1997). Das klassische Beispiel für die zentrale Stellung von GPCRs in der pharmakologischen Behandlung von Asthma ist der β₂AR (β₂-adrenerger Rezeptor) oder die Beteiligung eines Kaposi Sarkom (KS) assoziierten Herpesvirus GPCRs an der KS-induzierten Tumorgenese (Geras-Raaka et al., 1998). Diese Klasse von Rezeptoren binden ein breites Spektrum von Liganden, wie zum Beispiel Proteinhormone, Chemokine, Peptide oder divalente Kationen. Die Identifizierung von Aktivatoren und Inhibitoren G-Protein gekoppelter Rezeptoren ist somit einer der vielversprechendsten Ansätze zum besseren Verständnis und Behandeln von Krankheiten (Wilson et al., 1998).

Viele Komponenten des GPCR Signaltransduktionsweges in Säugerzellen haben orthologe Komponenten in dem GPCR Signaltransduktionsweg der Bäckerhefe Saccharomyces cerevisiae. Durch Stimulation mit Mating Faktor (Phermon α bzw. Phermon a) wird z. B. die Pheromon-abhängige Mitogen-aktivierte Proteinkinasekasdade aktiviert (im weiteren MAPK Kaskade genannt) (Frederickson, 1999). Diese Eigenschaft der Hefe kann dazu genutzt werden Säuger-GPCRs, im besonderen humane GPCRs, in einem Hefemodell zu testen, um nach Aktivatoren bzw. Inhibitoren des betreffenden GPCRs bzw. des entsprechenden Signaltransduktionsweges zu suchen, insbesondere, da speziesfremde GPCRs, in der Hefe funktionell exprimiert werden können, wobei es über die Hefe MAPK-Kaskade zu einer meßbaren zellulären Antwort kommt, wenn der eingeführte GPCR durch einen spezifischen Liganden aktiviert wird und dieses Signal an die MAPK Kaskade der Hefe weitergegeben wird. Von besonderem Interesse ist die Suche nach Aktivatoren bzw. Inhibitoren von bisher nicht funktionell annotierten GPCRs, den sogenannten Orphan-GPCRs.

Die Pheromone α oder a wirken in S. cerevisiae über die endogenen G-Protein gekoppelten Rezeptoren Ste2p und Ste3p (Gustin et al., 1998). Dabei wirkt das Pheromon α direkt auf die Pheromon-abhängige MAPK Kaskade, wodurch die Expression bestimmter S. cerevisiae Gene reguliert wird. Der Nachweis, ob eine Substanz eine aktivierende oder inhibierende Wirkung auf einen heterolog in S. cerevisiae exprimierten GPCR hat, kann durch die Expression S. cerevisiae Gene erfolgen, die durch die MAPK Kaskade reguliert werden ("Markergene"). Die Promotoren solcher Markergene können z.B. in Verbindung mit einem geeigneten Reportersystem zur Identifizierung von GPCR Aktivatoren bzw. Inhibitoren verwendet werden.

Die Identifizierung von Pheromon regulierten Genen kann einerseits über die Anwendung der Transposonmutagenese (Ross-Macdonald et al., 1999) oder die Anwendung von DNA-Mikrochips, die eine Expressionsanalyse für alle mRNAs einer Zelle zu einem bestimmten Zeitpunkt zuläßt, erfolgen (Wodicka et al., 1997).

Funktionelle Assays zur Identifizierung von Inhibitoren oder Aktivatoren von GPCRs werden bisher überwiegend in Säugerzellen durchgeführt (Wilson et al. (1998) British Journal of Pharmacology 125, 1387-1392); Geras-Raaka et al. (1998) J. Exp. Med. 188 No.2, 405-408). Für funktionelle Assays in S. cerevisiae ist die Verwendung der Promotoren des Gens FUS1 oder FUS2 beschrieben (Cisnowski et al. (1999) Nature 17,878-883; Frederickson (1999) Nature Biotechnology A, 852-853). FUS1 ist ein Gen, das nach Aktivierung mit Pheromon α in Wildtypzellen der Hefe Saccharomyces cerevisae erhöht exprimiert wird. In US 5,063,153 werden die Promotoren von FUS1 und FUS2 verwendet, um ein Strukturgen, das für ein Protein von Interesse kodiert, in großen Mengen zu exprimieren.
Eine Aufgabe der vorliegenden Erfindung war es, einen anderen S. cerevisiae Promotor zu identifizieren, der durch Pheromon a aktivierbar ist und mit dem eine starke Expression des regulierten Gens erreicht werden kann.

Gegenstand der Erfindung ist der Promotor des Gens YNL 279 w aus S. cerevisiae mit der Sequenz SEQ ID NO. 4.

Gegenstand der Erfindung ist auch ein rekombinantes DNA Fragment, das aus dem Promotor des Saccharomyces cerevisiae Gens YNL 279 w besteht, der, wenn funktionell mit einem Strukturgen verbunden, dessen Transkription reguliert und die DNA-Sequenz SEQ ID NO. 4 aufweist.
Gegenstand der Erfindung ist ein rekombinantes DNA Fragment, das den Promotor YNL 279 w und ein Strukturgen enthält und bei dem der Promotor funktionell mit dem Strukturgen verbunden ist Das Strukturgen kodiert beispielsweise für ein Reportergen oder für ein Protein, das in großen Mengen in S. cerevisiae hergestellt werden soll.

Gegenstand der Erfindung sind ein DNA-Vektor und eine rekombinante S.cerevisiae Zelle, die ein solches rekombinantes DNA-Fragment enthalten. Eine solche rekombinante S. cerevisiae Zelle exprimiert vorzugsweise keine oder nur geringe Mengen funktionell aktiver endogener Rezeptoren Ste2p und Ste3p.

Gegenstand der Erfindung sind auch Verfahren zur funktionellen Charakterisierung von GPCRs, zum Screening von Inhibitoren und/oder Aktivatoren von GPCRs und Verfahren zur Herstellung von Proteinen in S. cerevisiae.

Beispielsweise Verfahren zur Identifizierung von Aktivatoren und/oder Inhibitoren von G-Protein -gekoppelten Rezeptoren, wobei
a) eine rekombinante S. cerevisiae Zelle hergestellt wird, die ein Reportergen unter der Kontrolle des Promotors des YNL 279 w Gens enthält und die einen heterologen G-Protein gekoppelten Rezeptor exprimiert;
b) die Zelle mit einer zu untersuchenden Substanz inkubiert wird und
c) die Veränderung der Transkription des Reportergens bestimmt wird.

Verfahren zur Identifizierung von konstitutiv aktiven Mutanten von G-Protein gekoppelten Rezeptoren, wobei
a) eine rekombinante S, cerevisiae Zelle hergestellt wird, die ein oder mehrere Reportergene unter der Kontrolle des Promotors des YNL279w Gens enthält
b) und die einen mutierten heterologen G-Protein gekoppelten Rezeptor exprimiert, wobei die Modifikation zu einem konstitutiv aktiven G-Protein gekoppelten Rezeptor führt,
c) die Zelle mit einer aktivierenden oder inhibierenden Substanz inkubiert wird und
d) die Veränderung der Transkription des Reportergens bestimmt wird.

Vorzugsweise werden in der rekombinanten S. cerevisiae Zelle die entsprechenden endogenen GPCRs, Step2 und/oder Step3, nicht exprimiert, z.B. da diese Gene deletiert wurden. Diese Deletion kann z.B. wie in Broach Thomer (1996) Nature, 384, 14-16) beschrieben, durchgeführt werden.

Eine andere Ausführungsforrn betrifft Verfahren zur Herstellung von heterologen Proteinen in S. cerevisiae, wobei das Strukturgen des zu exprimierenden heterologen Proteins funktionell mit dem Promotor des YNL 279 w Gens verbunden und unter dessen Kontrolle exprimiert wird.

### Beschreibung der Erfindung im Einzelnen:

Zur Identifizierung Pheromon-induzierbarer und -reprimierbarer ORFs (Open-Reading-Frames, offene Leseraster) wurden Kulturen der Bäckerhefe Saccharomyces cerevisiae mit bzw. ohne Pheromon α behandelt um festzustellen, welche bekannten Gene und insbesondere bisher noch nicht charakterisierten Gene durch Pheromon α induziert bzw. reprimiert werden. Es wurden DNA-Mikrochips verwendet, mit denen parallel die Expressionsmuster für die mehr als 6000 identifizierten Hefegene dargestellt werden können. Vergleicht man die Genexpressionsmuster von zwei Zellen, bzw. Kulturen von Zellen, die in unterschiedlicher Weise z.B. mit oder ohne Pheromon α behandelt wurden, so läßt sich mit Hilfe der DNA-Mikroarrays, nach entsprechender Aufbereitung der korrespondierenden mRNAs, feststellen, welche Gene spezifische aktiviert, bzw. reprimiert wurden.

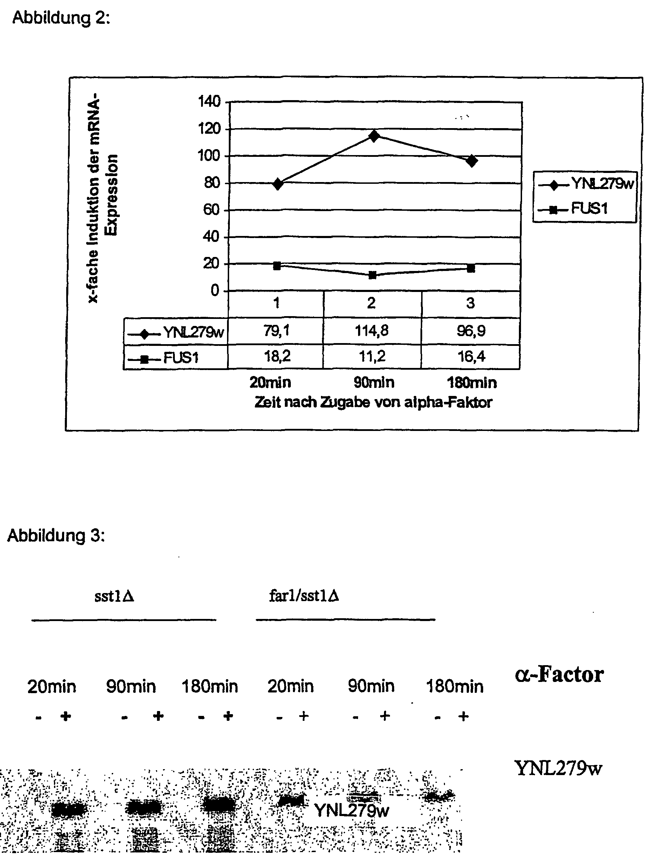

Der ORF YNL279w zeigte nach Pheromon α Zugabe zeitabhängig einen starken Anstieg seiner Expression (Abbildung 2 und 3). Da die Regulation der Transkription bzw. Expression von Genen in der Regel über regulative Sequenzen 5' vom Startpunkt der Translation, den sogenannten Promotoren, erfolgt, sind die Promotoren die solche regulativen Sequenzen besitzen von besonderem Interesse.
Zur Identifizierung von Aktivatoren bzw. Inhibitoren von pharmakologisch und medizinisch interessanten GPCRs können solche induzierbaren oder reprimierbaren Promotoren in gleicher Weise vor Gene bzw. Strukturgene kloniert werden, die ein geeignetes Reporter-System darstellen, um Substanzen zu identifizieren, die nach Zugabe aktivierende bzw. inhibierende Wirkung auf das Reporter System zeigen. Geeignete Reporter-Systeme stellen beispielsweise die Gene von LacZ, Luciferase, Aequorin, Green-fluorescent Protein (GFP), DsRed, HIS3, URA3, TRP1 und LEU2, aber auch Resistenzgene gegen bestimmte Antibiotika, z. B. Kanamycin dar.
Bei diesen sogenannten Reportergenen kann es sich z.B. um Gene wie z.B. lacZ, handeln, bei denen über Verfärbung der Zellkolonien eine Wirkung bestimmter Substanzen auf die MAPK Kaskade nachgewiesen werden kann. Bei den Reportergenen kann es sich auch z.B. um Gene handeln, die der Zelle unter bestimmten Selektionsbedingungen das Wachstum ermöglichen, wie z.B. auxotrophe Gene.

Beispiele für rekombinante DNA Fragmente (DNA Konstrukte), die den Promotor des Gens YNL279w und dahinter klonierte Reportergene enthalten, sind in Tabelle 1 dargestellt. Diese DNA Konstrukte können z.B. für die Identifizierung von GPCR-Aktivatoren (Agonisten) und Inhibitoren (Antagonisten) in funktionellen Assays verwendet werden.

**Tabelle 1:**

| DNA Konstrukt | Nachweissystem (Read out) |
|---|---|
| Promotor(YNL279w)-LacZ | kolorimetrischer/Chemiluminiszenz Read-out |
| Promotor(YNL279w)-Luziferase | Chemiluminiszenz Read-out |
| Promotor(YNL279w)-Aeqourin | Chemiluminiszenz Read-out |
| Promotor(YNL279w)-GFP | Fluoreszierende Kolonien (Zellen) |
| Promotor(YNL279w)-EGFP | Fluoreszierende Kolonien (Zellen) |
| Promotor(YNL279w)-KAN | Wachstum in Selektionsmedium |
| Promotor(YNL279w)-HlS3 | Wachstum in Histidin-depletiertem Medium |
| Promotor(YNL279w)-URA3 | Wachstum in Uracil-depletiertem Medium |
| Promotor(YNL279w)-TRP1 | Wachstum in Tryptophan-depletiertem Medium |
| Promotor(YNL279w)-LEU2 | Wachstum in Leucin-depletiertem Medium |
| Promotor(YNL279w)-ADE2 | Wachstum in Adenin-depletiertem Medium |
| Promotor(YNL279w)-CAN1 | Wachstum in Canavanin-haltigem Medium (bei Zugabe eines Inhibitors) |

Die DNA, welche für ein solches Reportergen codiert, wird 3' an den Pheromoninduzierbaren Promotor des ORFs YNL279w der Hefe Saccharomyces cerevisae fusioniert und z.B. in einen high-copy Vektor oder low-copy Vektor kloniert. Mit diesen Vektoren können dann Hefezellen transformiert werden. Ebenso kann die an den Promotor fusionierte DNA stabil in das Hefegenom integriert werden.

Hefestämme, die mit Reportergenen genetisch manipuliert werden, die zur Gruppe der auxotrophen Gene (z.B. HIS3, URA3, TRP1, LEU2, ADE2 und LYS1) gehören, müssen für das korrespondierende Gene mutiert, d.h. funktionell inaktiviert sein.

Bei Stimulation der Hefezellen durch Pheromon kommt es zu einer Aktivierung des Hefepromotors YNL 279 w und zur erhöhten Expression des durch den Promotor regulierten Reportergens. Bei der Suche nach GPCR-Agonisten wird der Promotor des Gens YNL279w vorzugsweise vor Reportergene wie z.B. HIS, ADE, TRP oder LEU kloniert, da diese Kombination einen Read-out über das Wachstum der Zellen in einem entsprechend depletiertem Medium zuläßt. Wird ein ausgewählter GPCR über eine zu untersuchende Substanz aktiviert, kommt es zur Aktivierung des am Ende der MAPK-Kaskade stehenden Promotors, YNL279w, und somit zur Expression durch den YNL 279 w Promotor regulierten Reportergens. Im Falle von HIS, ADE, TRP oder LEU führt dieses zu einem Wachstum im entsprechenden Selektionsmedium. Ein Agonist kann dadurch identifiziert werden, daß die entsprechenden S. cerevisiae Zellen in dem Selektionsmedium wachsen.

Bei Verwendung der Gene für LacZ, GFP oder EGFP kann ein Read-out über kolorimetrische, bzw. luminometrische Messungen erfolgen. Nur solche Zellen zeigen eine Chemiluminiszenz, bei denen der heterologe GPCR durch einen Agonisten aktiviert wurde.

Wird als Reportergen CAN1 verwendet, wachsen die Zellen in Canavanin-haltigem Medium. In Gegenwart von Aktivatoren (Agonisten) eines heterolog exprimierten GPCRs wird das CAN1-Gen exprimiert, so daß die Zellen nicht mehr in dem Canavanin-haltigen Medium wachsen. Wird ein Antagonist (Inhibitor) hinzugefügt, so wachsen die Kulturen in diesem Selektionsmedium.

Die Screening-Verfahren können auf Mikrotiterplatten durchgeführt werden, in denen Hefezellen ausgesät wurden, die zuvor mit der Promotor-Reportergen-Konstrukt (z.B. ein DNA-Konstrukt aus Tabelle 1) transformiert wurden. Zusätzlich werden diese Zellen mit einem heterologen GPCR transformiert, für den ein Agonist bzw. Antagonist gefunden werden soll.
Beispielsweise können humane GPCRs, z.B. Adenosin-Rezeptoren, Somatostatin-Rezeptoren, Dopamine-Rezeptoren, Bradykinin-Rezeptoren, Lysolipid-Rezeptoren, β-Adrenerge-Rezeptoren, Muscarinische Acetylcholin-Rezeptoren in diesen Verfahren verwendet werden.

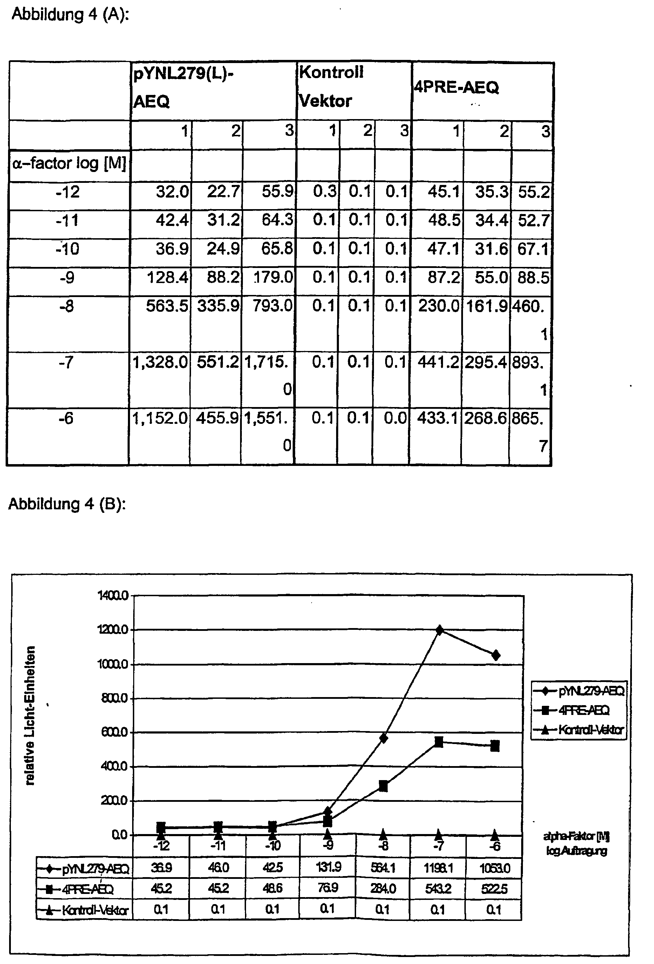

Als ein Pheromon α induziertes Gen kann der Promotor von YNL279w auch zur Identifizierung von Aktivatoren und Inhibitoren der Pheromon-abhängigen MAPK Kaskade verwendet werden. Da der Promotor YNL279w sensitiver auf Stimulation nach Pheromon α Behandlung reagiert, als dieses beispielsweise für den FUS1-Promotor der Fall ist, bietet die hier vorgeschlagene Verwendung des YNL279w-Promotors Vorteile gegenüber anderen Screeningverfahren, die z.B. den FUS1 Promotor verwenden. Abbildung 4 zeigt, daß die Stimulation des YNL279w-Promotor/Aeqourin-Konstruktes (p415YNL279-AEQ) deutlich besser reagiert, als dies für ein Promotorelement der Fall ist, das einem Bereich aus dem Promotor des FUS1-Gens (hier als 4PRE-AEQ bezeichnet) entspricht.

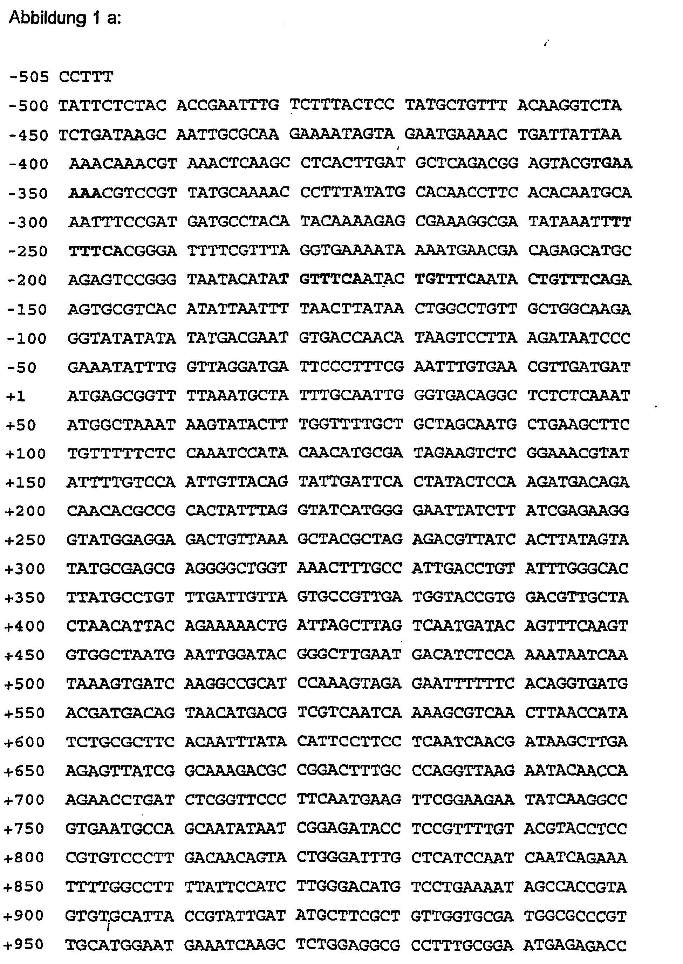

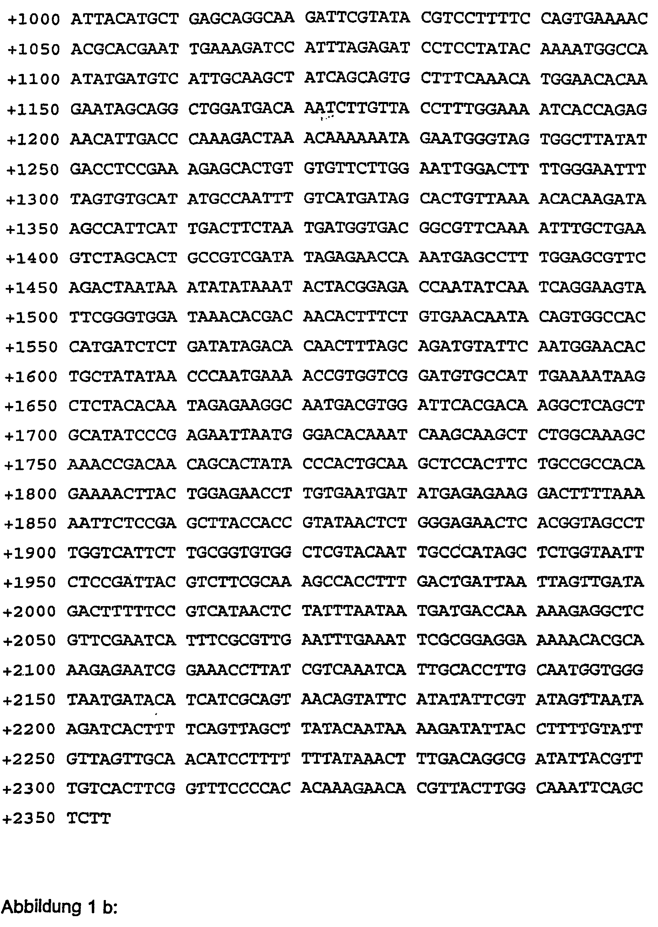

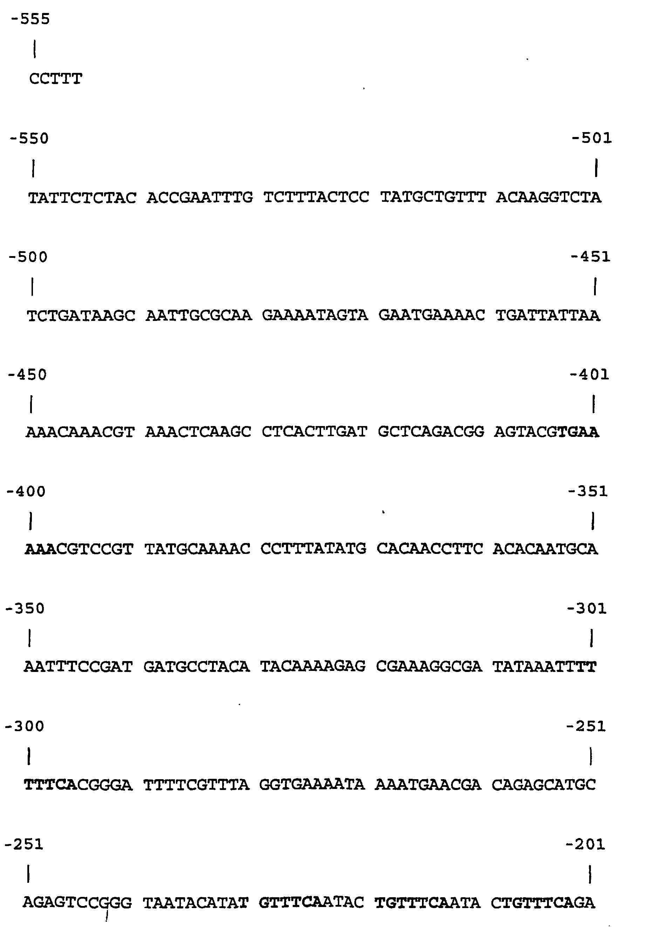

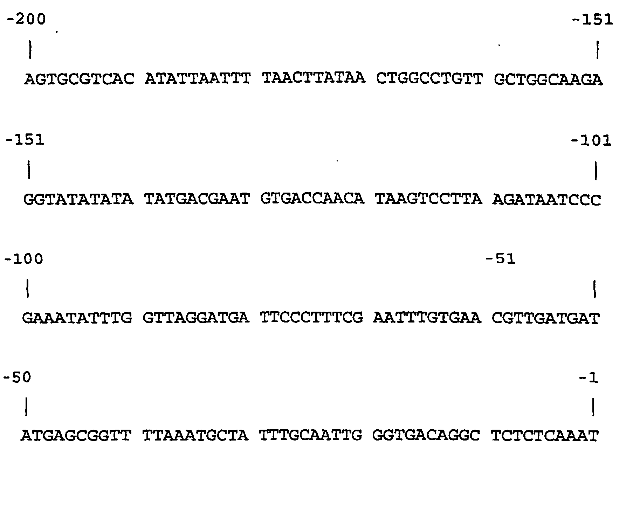

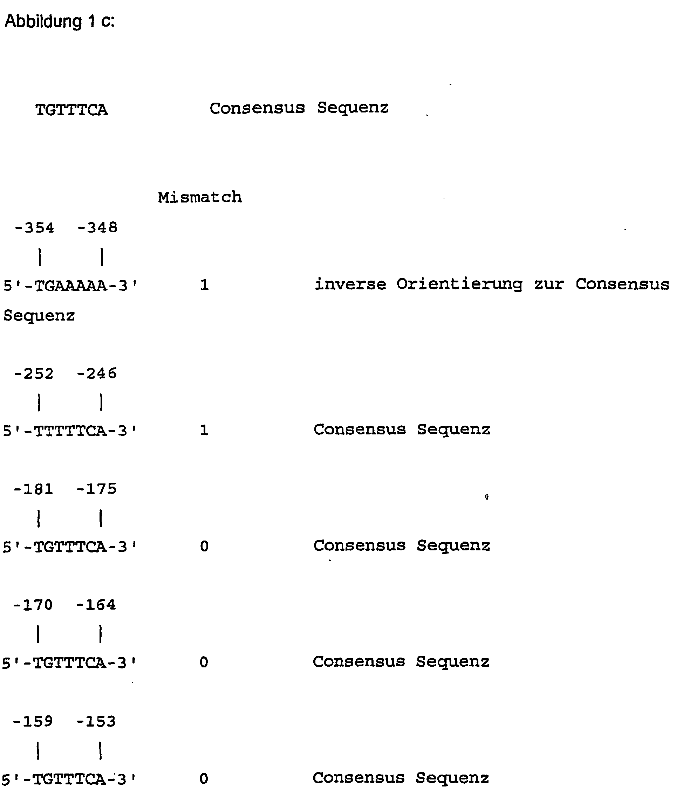

Abbildung 1a: Sequenz von YNL279w (Genbank AccNr.:Z71555, SEQ ID NO. 3). Das Startcodon ATG ist unterstrichen. Fett unterlegte Sequenzbereiche stellen Pheromone responsive elements (PRE) dar.
Abbildung 1b: Promotorregion (5'-UTR) von YNL279w (SEQ ID NO. 4)
Abbildung 1c: Pheromone responsive elements (PRE) im Promotorbereich von YNL279w
Abbildung 2: Expressionsprofil des Saccharomyces cerevisiae ORFs YNL279w nach Induktion mit Pheromon α. Die beobachteten Änderungen der Genexpression beruhen auf Ergebnissen der Untersuchungen mit DNA-Mikrochips. Die Änderungen der Genexpression des Gens FUS1 sind zum Vergleich dargestellt.
Abbildung 3: Northem Blot Analyse des Expressionsprofils des Gens YNL279w nach Stimulation der Hefekulturen mit Pheromon α in der Hefemutante sst1.
Abbildung 4: Induktion der Aequorin Expression (als relative Lichteinheiten) nach Stimulation der Hefezellen mit den angegebenen Konzentrationen Pheromon α (B). Die im Graphen angegebenen Werte stellen den Mittelwert für drei unabhängig durchgeführte Versuche dar, die dazugehörigen Einzelwerte sind in (A) dargestellt Das Aequorin-Reportergen steht bei pYNL279-AEQ unter der Kontrolle des Promotors von YNL279w. Bei 4PRE-AEQ steht Aequorin unter Kontrolle eines Bereiches (-271 bis -1 vor Start) des Gens FUS1. Jeder Stimülationsversuch wurde dreifach durchgeführt.
Abbildung 5: Schematische Darstellung des Promotor-Reportergen-Vektor-Konstruktes, bestehend aus dem Promotorbereich von YNL279w und dem Reportergen Aeqourin in dem Vektor p415 (einem Derivat von Vektor pRS415: ATCC 87520).

### Beispiele

### Beispiel 1: Identifizierung des ORF YN279w

Zellenkulturen des Hefestammes Saccharomyces cerevisiae mit dem Genotyp (MAT a sst1::LEU2; ade2;can1-100; his3-11,15; leu2-3,112; trp1-1; ura3-1) wurden mit 1µM Pheromon α (gelöst in 90% Methanol) stimuliert, Kontrollzellen wurden nur mit 90% Methanol behandelt. 20min, 90min und 180min nach Stimulation wurden eine Menge von Zellen entnommen, die etwa 10 OD₆₀₀ₙₘ entspricht. Gesamt-RNA aus diesen Zellen wurde nach Standardmethoden (z.B. Sambrock et al. Molecular Chemistry Press, (Cold Spring Harbor Laboratory 1989) isoliert und mit je 30µg Gesamt-RNA wurde eine Erststrangsynthese durchgeführt. Das Protokoll für die Erst- und Zweitstrangsynthese, wie auch die Aufreinigungsschritte der generierten doppelsträngigen cDNA, die in-vitro Transcription und die Aufbereitung der cRNA für die Hybridisierung entspricht dem Protokoll der Firma Affymetrix Inc. (Santa Clara, CA, USA).

Über den Vergleich der Expressionsprofile mit Pheromon α induzierter und nichtinduzierter Hefezellen wurden der ORF YNL279w als einer der am stärksten induzierten ORFs identifiziert.
Zur Feststellung der Aktivität und Verwendbarkeit des Promotors in einem Screeningsystem mit einem geeigneten Reporter wurden der YNL279w Promotorbereich aus genomischer Saccharomyces cerevisiae-DNA amplifiziert.

### Beispiel 2: Isolierung des YNL279w Promotors ("Promotor")

Der Promotorbereich des Gens YNL279w wurde über die Primer YNL279F1 (SEQ ID NO. 1; 5'-CCGAGTCCTACTCCTATGCTGTTTACAAGG-3') und YNL279R (SEQ ID NO. 2; 5'-TGCTCTAGAATCATCAACGTTCACAAATTCG-3') amplifiziert.
Die Identität des resultierenden Amplifikates wurde über
Standardsequenzierungsmethoden festgestellt.
Länge des über YNL279F1/YNL279R amplifizierten Promotorbereiches ohne Restriktionsschnittstellen beträgt: 473 bp; dieses Fragment des Promotorbereichs wird im weiteren "Promotor" genannt.

### Beispiel 3: Bestimmung der Funktionalität

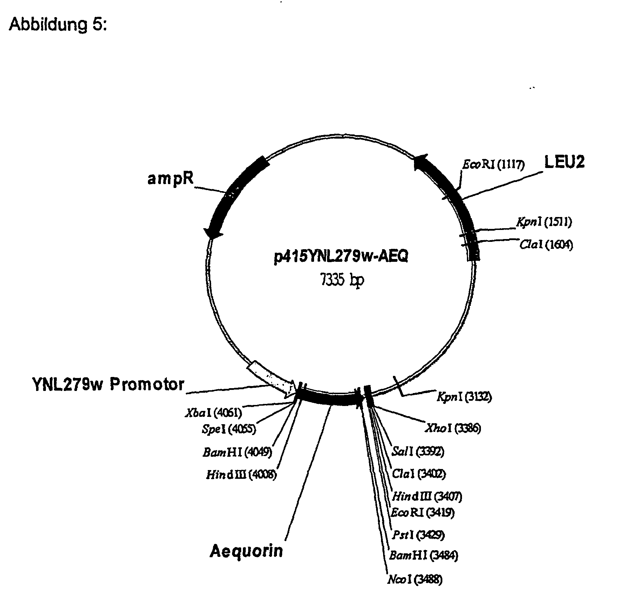

Zur Feststellung der Funktionalität des vorgeschlagenen Screeningverfahrens wurde der amplifizierte Promotorbereich des Gens YNL279w vor das für Aequorin kodierende Gen in den Vektor p415 (siehe Abbildung 5; Derivat des Vektors pRS415, ATCC 87520) kloniert. Dieses Konstrukt wurde über die Li-Acetat-Methode (Ito et al., 1983) in den Saccharomyces cerevisiae Stamm (Mat a far1::hisG sst2::ura3^{FOA} fus1::HIS3) transformiert.
Die Detektion von Aeqourin nach Stimulation der MAPK Kaskade mit Pheromone α in in den Saccharomyces cerevisiae Stamm (Mat a far1::hisG sst2::ura3^{FOA} fus1::HIS3) erfolgte wie dargestellt:
1. 25µl Zellsuspension (1,5.10⁶ Zellen per 25µl in selektivem Medium, SC/Glucose-Leucin) wurden in eine Vertiefung einer 96-Loch Platte gegeben
2. Auf die Zellen wurden 25µl eines 2X konzentrierten Stimulationsmixes gegeben, der Mating Faktor α in serieller Verdünnung (10⁻⁶ M to 10⁻⁸ M) und 1 µM Coelenterazin (0,5µM Endkonzentration) enthielt
3. 96-Lochplatte vorsichtig schütteln
4. 96-Loch Platte für 1,5 Stunden in feuchter Atmosphäre bei 30°C im Dunkeln inkubieren
5. Zugabe von 150µl Calcium/Lysepuffer zur Detektion; Aequorin emitiert Photonen direkt nach der Zugabe dieses Mixes, 15s Integration des entstehenden Signals; Detektion des Signals mittels eines Luminometers (Luminoskan, LABSYSTEM)

Mating faktor α Stocklösung: 100µM in 90% Methanol
Coelenterazin Stocklösung: 1 mM in 100% Methanol (MOLECULAR PROBES; ref C-2944).
CaCl₂ Stocklösung: 1 M in 1 M Tris-HCl Lösung (vor Gebrauch 100 fach verdünnt in Lysepuffer)
Lysepuffer: Yeast Protein Extraction Reagent; PIERCE, ref 78990

### Referenzen

Cismowski MJ, Takesono A, Ma C, Lizano JS, Xie X, Fuemkranz H, Lanier SM, Duzic E. (1999) Genetic screens in yeast to identify mammalian nonreceptor modulators of G-protein signaling. Nat Biotechnol 17:878-83

Erdman S, Lin L, Malczynski M, Snyder M. (1998) Pheromone-regulated genes required for yeast mating differentiation. J Cell Biol 140:461-83

Fink, GR, Trueheart, J, Elion, EA. (1991) Pheromone-inducible yeast promoter. United States Patent 5.063.154

Frederickson RM. (1999) Budding actors in mammalian G-protein signaling. Nat Biotechnol 17:852-3

Geras-Raaka E, Varma A, Ho H, Clark-Lewis I, Gershengom MC. (1998) Human interferon-gamma-inducible protein 10 (IP-10) inhibits constitutive signaling of Kaposi's sarcoma-associated herpesvirus G
protein-coupled receptor. J Exp Med 188:405-8

Gustin MC, Albertyn J, Alexander M, Davenport K. (1998) MAP kinase pathways in the yeast Saccharomyces cerevisiae. Microbiol Mol Biol Rev 62:1264-300

Stadel JM, Wilson, S, Bergsma, DJ. (1997) Orpahn G protein-coupeld receptors: a neglected opportunity for pioneer drug discovery. TiPS 18:430-437

Wilson S, Bergsma DJ, Chambers JK, Muir Al, Fantom KG, Ellis C, Murdock PR, Herrity NC, Stadel JM (1998) Orphan G-protein-coupled receptors: the next generation of drug targets? Br J Pharmacol 125:1387-92

Wodicka L, Dong H, Mittmann M, Ho MH, Lockhart DJ. (1997) Genome-wide expression monitoring in Saccharomyces cerevisiae. Nat Biotechnol 15:1359-67

lto H, Fukuda Y, Murata K, Kimura A (1983) Transformation of intact yeast cells treated with alkali cations. J Bacteriol Jan;153:163-8

Ross-Macdonald P, Coelho PS, Roemer T, Agarwal S, Kumar A, Jansen R, Cheung KH, Sheehan A, Symoniatis D, Umansky L, Heidtman M, Nelson FK, lwasaki H, Hager K, Gerstein M, Miller P, Roeder GS, Snyder M (1999) Large-scale analysis of the yeast genome by transposon tagging and gene disruption. Nature 402:413-8

## Patentansprüche

1. Rekombinantes DNA Fragment bestehend aus dem Promotor mit der DNA-Sequenz

2. Rekombinantes DNA Fragment nach Anspruch 1, enthaltend weiterhin ein Strukturgen, wobei der Promotor funktionell mit dem Strukturgen verbunden ist und dessen Expression reguliert.

3. Rekombinantes DNA Fragment nach Anspruch 2, wobei das Strukturgen für ein Reportergen kodiert.

4. Rekombinantes DNA Fragment nach Anspruch 3, wobei das Reportergen ausgewählt wird aus LacZ, Luciferase, Aequorin, GFP, DsRed, His 3, URA 3, TRP1 oder LEU2.

5. DNA-Vektor enthaltend ein rekombinantes DNA-Fragment nach einem der Ansprüche 1 bis 4.

6. Rekombinante S. cerevisiae Zelle enthaltend ein rekombinantes DNA-Fragment oder einen Vektor nach einem der Ansprüche 1 bis 5.

7. Verfahren zur Identifizierung von Aktivatoren von G-Protein-gekoppelten Rezeptoren, wobei
a) eine rekombinante S. cerevisiae Zelle hergestellt wird, die ein oder mehrere Reportergene unter der Kontrolle des Promotors des YNL 279 w Gens gemäß Anspruch 1 enthält und die einen heterologen G-Protein gekoppelten Rezeptor exprimiert;
b) die Zelle mit einer zu untersuchenden Substanz inkubiert wird und
c) die Veränderung der Transkription des Reportergens bestimmt wird.

8. Verfahren zur Identifizierung von Inhibitoren von G-Protein-gekoppelten Rezeptoren, wobei
a) eine rekombinante S. cerevisiae Zelle hergestellt wird, die ein oder mehrere Reportergene unter der Kontrolle des Promotors des YNL 279 w Gens gemäß Auspruch 1 enthält und die einen heterologen G-Protein-gekoppelten Rezeptor exprimiert;
b) die Zelle mit einer zu untersuchenden Substanz inkubiert wird und
c) die Veränderung der Transkription des Reportergens bestimmt wird.

9. Verfahren zur Herstellung von heterologen Proteinen in S. cerevisiae, wobei das Strukturgen des heterologen Proteins funktionell mit dem Promotor des YNL 279w Gens gemäß Anspruch 1 verbunden und unter dessen Kontrolle exprimiert wird.

10. Verfahren zur Identifizierung von konstitutiv aktiven Mutanten von G-Protein gekoppelten Rezeptoren, wobei
a) eine rekombinante S. cerevisiae Zelle hergestellt wird, die ein oder mehrere Reportergene unter der Kontrolle des Promotors des YNL279w Gens gemäß Anspruch 1 enthält
b) und die einen mutierten heterologen G-Protein gekoppelten Rezeptor exprimiert, wobei die Modifikation zu einem konstitutiv aktiven G-Protein gekoppelten Rezeptor führt,
c) die Zelle mit einer aktivierenden oder inhibierenden Substanz inkubiert wird und
d) die Veränderung der Transkription des Reportergens bestimmt wird.

## Claims

1. A recombinant DNA fragment consisting of the promoter having the DNA sequence

2. The recombinant DNA fragment as claimed in claim 1, further comprising a structural gene, wherein the promoter is functionally linked to the structural gene and regulates expression thereof.

3. The recombinant DNA fragment as claimed in claim 2, wherein the structural gene codes for a reporter gene.

4. The recombinant DNA fragment as claimed in claim 3, wherein the reporter gene is selected from lacZ, luciferase, aequorin, GFP, dsRed, His 3, URA 3, TRP1 and LEU2.

5. A DNA vector comprising a recombinant DNA fragment as claimed in any of claims 1 to 4.

6. A recombinant S. cerevisiae cell comprising a recombinant DNA fragment or a vector as claimed in any of claims 1 to 5.

7. A method for identifying activators of G protein-coupled receptors, wherein
a) a recombinant S. cerevisiae cell is produced, which contains one or more reporter genes under the control of the promoter of the YNL 279 w gene as claimed in claim 1 and which expresses a heterologous G protein-coupled receptor;
b) the cell is incubated with a substance to be studied, and
c) the change in reporter gene transcription is determined.

8. A method for identifying inhibitors of G protein-coupled receptors, wherein
a) a recombinant S. cerevisiae cell is produced, which contains one or more reporter genes under the control of the promoter of the YNL 279 w gene as claimed in claim 1 and which expresses a heterologous G protein-coupled receptor;
b) the cell is incubated with a substance to be studied, and
c) the change in reporter gene transcription is determined.

9. A method for preparing heterologous proteins in S. cerevisiae, wherein the structural gene of the heterologous protein is functionally linked to the promoter of the YNL 279w gene as claimed in claim 1 and is expressed under the control thereof.

10. A method for identifying constitutively active mutants of G protein-coupled receptors, wherein
a) a recombinant S. cerevisiae cell is produced, which contains one or more reporter genes under the control of the promoter of the YNL279w gene as claimed in claim 1
b) and which expresses a mutated heterologous G protein-coupled receptor, the modification resulting in a constitutively active G protein-coupled receptor,
c) the cell is incubated wiith an activating or inhibiting substance, and
d) the change in reporter gene transcription is determined.

## Revendications

1. Fragment d'ADN recombinant constitué du promoteur de séquence d'ADN :

2. Fragment d'ADN recombinant selon la revendication 1, contenant en outre un gène structurel, dans lequel le promoteur est fonctionnellement lié au gène structurel et régule son expression.

3. Fragment d'ADN recombinant selon la revendication 2, dans lequel le gène structurel code pour un gène rapporteur.

4. Fragment d'ADN recombinant selon la revendication 3, dans lequel le gène rapporteur est choisi parmi les gènes LacZ, Luciférase, Aequorine, GFP, DsRed, His 3, URA3, TRP1 ou LEU2.

5. Vecteur d'ADN contenant un fragment d'ADN recombinant selon l'une quelconque des revendications 1 à 4.

6. Cellule recombinante de S. cerevisiae contenant un fragment d'ADN recombinant ou un vecteur selon l'une quelconque des revendications 1 à 5.

7. Procédé d'identification d'activateurs de récepteurs couplés à des protéines G, dans lequel
a) on fabrique une cellule recombinante de S. cerevisiae qui contient un ou plusieurs gènes rapporteurs sous le contrôle du promoteur du gène YNL 279 w selon la revendication 1 et qui exprime un récepteur hétérologue couplé à des protéines G;
b) on incube la cellule avec une substance à étudier; et
c) on détermine la variation de la transcription du gène rapporteur.

8. Procédé d'identification d'inhibiteurs de récepteurs couplés à des protéines G, dans lequel
a) on fabrique une cellule recombinante de S. cerevisiae qui contient un ou plusieurs gènes rapporteurs sous le contrôle du promoteur du gène YNL 279 w selon la revendication 1 et qui exprime un récepteur hétérologue couplé à des protéines G;
b) on incube la cellule avec une substance à étudier; et
c) on détermine la variation de la transcription du gène rapporteur.

9. Procédé de fabrication de protéines hétérologues dans S. cerevisiae, dans lequel le gène structurel de 1a protéine hétérologue est fonctionnellement lié avec le promoteur du gène YNL 279 w selon la revendication 1 et est exprimé sous son contrôle.

10. Procédé d'identification de mutants, constitutivement actifs, de récepteurs couplés à des protéines G, dans lequel
a) on fabrique une cellule recombinante de S. cerevisiae, qui contient un ou plusieurs gènes rapporteurs sous le contrôle du promoteur du gène YNL 279 w selon la revendication 1 et
b) qui exprime un récepteur hétérologue couplé à des protéines G, muté, dans lequel la modification conduit à un récepteur constitutivement actif couplé à des protéines G,
c) on incube la cellule avec une substance activante ou inhibitrice et
d) on détermine la variation de la transcription du gène rapporteur.
